# EUROPÄISCHE PATENTSCHRIFT

(11) **EP 0 619 287 B1**
(45) Veröffentlichungstag und Bekanntmachung des Hinweises auf die Patenterteilung: **25.06.1997**
(21) Anmeldenummer: 94104837.3
(22) Anmeldetag: 26.03.1994
(51) Int. Cl.: C07C 17/38, C07C 17/10, C07C 19/08

(54) **Herstellung von Perfluoralkanen**
Preparation of perfluoralkanes
Préparation de perfluoroalkanes

(30) Priorität: 03.04.1993 DE 4311062
(43) Veröffentlichungstag der Anmeldung: 12.10.1994
(73) Patentinhaber: Solvay Fluor und Derivate GmbH, D-30173 Hannover (DE)
(72) Erfinder: Herkelmann, Ralf, D-30457 Hannover (DE); Rudolph, Werner, D-30559 Hannover (DE); Sander, Rüdiger, D-31319 Sehnde (DE); Schulz, Alf, D-31535 Neustadt (DE)
(74) Vertreter: Lauer, Dieter, Dr.

(56) Entgegenhaltungen:
- US-A- 2 496 115
- US-A- 3 686 082

## Beschreibung

Die Erfindung bezieht sich auf ein Verfahren zur Herstellung von Perfluoralkanen durch Umsetzung von Gemischen, die Perfluoralkane und Polyfluoralkane enthalten, mit elementarem Fluor unter Druck bei erhöhter Temperatur.

Perfluorierte organische Verbindungen sind chemisch sehr stabil und nicht brennbar, nicht toxisch und geruchlos. Sie werden aufgrund des hohen Sauerstofflösevermögens u. a. als Blutersatzstoff verwendet. Sie eignen sich für andere Zwecke, z. B. als Wärmeträgerflüssigkeit oder als Lösungsmittel. Dabei ist es vorteilhaft, wenn das perfluorierte organische Material möglichst frei von polyfluorierten Verbindungen ist, da polyfluorierte Verbindungen toxisch sein können und nicht die gewünschte Stabilität perfluorierter organischer Verbindungen aufweisen.

Zur Herstellung perfluorierter Verbindungen sind verschiedene Verfahren bekannt. Gut geeignet sind beispielsweise Methoden, die auf dem Austausch von Wasserstoffatomen gegen Fluoratome beruhen, z. B. die Elektrofluorierung, die Fluorierung mit Metallfluoriden wie AgF₂, oder die Umsetzung mit elementarem Fluor. Dabei kann man jeweils von nichtfluorierten oder teilfluorierten Verbindungen ausgehen.

Zur Reinigung von Gemischen, die bei solchen Verfahren erhalten werden und perfluorierte sowie polyfluorierte organische Verbindungen aufweisen, gibt es verschiedene Methoden: Mit Verlust an Material ist die Umsetzung der Gemische mit starken Basen verbunden, da dabei die polyfluorierten Verbindungen zerstört und abgetrennt werden. Besser sind Verfahren, in welchen die polyfluorierten Verbindungen in perfluorierte Verbindungen überführt werden. Die US-A 4,220,606 beschreibt beispielsweise ein Verfahren, bei welchem Kohlenwasserstoffe in drei Stufen unter zunehmend drastischeren Bedingungen mit Silberfluorid, Kobaltfluorid oder Schwefelfluoriden umgesetzt werden. Dabei kann man in der dritten Stufe auch von teilfluoriertem Material ausgehen, das auf andere Weise erhalten wurde. Das Verfahren ist recht umständlich. Die US-A 2,496,115 beschreibt ein Verfahren zur Stabilisierung von Perfluorcarbonen, bei welchem ein Gemisch von perfluorierten und polyfluorierten Verbindungen mit elementarem Fluor behandelt wird, um polyfluorierte Verbindungen in perfluorierte Verbindungen zu überführen. Analog geht man bei dem in der EP-A 271 272 beschrieben Verfahren vor. Dabei wird Fluor, das gegebenenfalls mit Inertgas wie Stickstoff verdünnt wird, durch das zu behandelnde Gemisch geleitet. In der US-A 2,496,115 wird in Beispiel 1 geschildert, daß nur dann eine vollständige Perfluorierung gelingt, wenn man die Reaktionstemperatur von zunächst 150 °C auf 300 °C steigert. Unter diesen Bedingungen liegt die Ausbeute bei lediglich 78 %.

Aufgabe der vorliegenden Erfindung ist es, ein Verfahren anzugeben, mit welchem sich polyfluorierte Verbindungen, die im Gemisch mit perfluorierten Verbindungen vorliegen, mit hoher Ausbeute und hoher Reaktionsgeschwindigkeit in perfluorierte Verbindungen umwandeln lassen. Diese Aufgabe wird durch die vorliegende Erfindung gelöst. Die Erfindung beruht auf der Erkenntnis, daß bei erhöhtem Druck eine Fluorierung von polyfluorierten Verbindungen mit elementarem Fluor mit hoher Ausbeute, aber geringer Tendenz zur Bildung von Nebenprodukten möglich ist.

Das erfindungsgemäße Verfahren zur Herstellung von Perfluoralkanen durch Umsetzung von Gemischen, die Perfluoralkane und Polyfluoralkane enthalten, mit einem Reaktivgas, ausgewählt aus der Gruppe umfassend elementares Fluor und ein Fluor/Inertgas-Gemisch ist dadurch gekennzeichnet, daß man ein Reaktivgas mit einem Gehalt von 40 bis 100 Vol.-% Fluor und 0 bis 60 Vol.-% Inertgas einsetzt und die Umsetzung bei einem Druck von 4 bis 12 bar (abs.) und einer Temperatur von 75 bis 120 °C durchführt. Vorzugsweise sind im hergestellten Produkt weniger als 100 ppm an Polyfluoralkanen enthalten. Eine überraschende Eigenschaft des erfindungsgemäßen Verfahrens besteht zunächst darin, daß man viel mehr Fluor einsetzen kann, als theoretisch zum vollständigen Austausch der Wasserstoffatome gegen Fluoratome notwendig ist. Dennoch beobachtet man allenfalls eine geringe Bildung von Nebenprodukten oder Zersetzungsprodukten, beispielsweise durch Spaltung von Kohlenstoff-Kohlenstoff-Bindungen. Die Wirksamkeit des Verfahrens bei der Stabilisierung oder Reinigung von Perfluoralkanen ist sehr hoch.

Im Rahmen der vorliegenden Erfindung bedeutet der Begriff "polyfluorierte" Verbindungen, polyfluorierte Alkane. Vorzugsweise beträgt das F/H-Verhältnis 3:1 oder höher. Bevorzugt bedeutet der Begriff "polyfluorierte Verbindungen" lineare oder verzweigte polyfluorierte aliphatische Alkane mit 3 bis 18 C-Atomen mit dem angegebenen F/H-Mindestverhältnis, und der Begriff "perfluorierte Verbindungen" bedeutet lineare oder verzweigte perfluorierte aliphatische Alkane mit 3 bis 18 C-Atomen. Natürlich kann man auch Gemische solcher Alkane behandeln. Bevorzugtes Inertgas ist N₂.

Insbesondere setzt man Gemische ein, die neben perfluorierten Verbindungen maximal 0,5 Mol.-% an Polyfluoralkanen enthalten.

Es hat sich als vorteilhaft erwiesen, die Umsetzung mit einem Reaktivgas umfassend 40 bis 60 Vol.-% Fluor und 60 bis 40 Vol.-% Inertgas sowie bei einem Druck von 5 bis 10 bar (abs.) und einer Temperatur von 80 bis 120 °C durchzuführen. Bevorzugt führt man die Umsetzung bei einer Temperatur von 80 bis 100 °C durch.

Beim Austausch von Wasserstoff gegen Fluor nach dem erfindungsgemäßen Verfahren wird HF freigesetzt. Der gebildete Fluorwasserstoff kann bei der Aufarbeitung des Reaktionsprodukts nach üblichen Methoden abgetrennt werden. Bevorzugt absorbiert man mittels Kaliumfluorid oder Natriumfluorid. Besonders vorteilhaft wird das Verfahren so durchgeführt, daß man den Wasserstoff-Fluor-Austausch in Anwesenheit einer zur Adsorption des gebildeten Fluorwasserstoffs ausreichenden Menge eines HF-Adsorptionsmittels, vorzugsweise KF oder NaF, durchführt.

Die Zeitdauer, während welcher man das erfindungsgemäße Verfahren durchführt, ist vom gewünschten Reinigungsgrad sowie vom Gehalt an polyfluorierten Verbindungen, sowie auch vom angewendeten Druck und der angewendeten Temperatur und der relativen Menge an elementarem Fluor abhängig. Optimale Bedingungen kann man durch entsprechende Handversuche ermitteln. Vorteilhaft führt man das erfindungsgemäße Verfahren während einer Zeitdauer von 5 bis 10 Stunden durch.

Das erfindungsgemäße Verfahren eignet sich besonders gut zur Herstellung bzw. zur Stabilisierung oder Reinigung von linearen oder verzweigten, aliphatischen Perfluoralkanen mit 5 bis 7 C-Atomen.

Nach dem erfindungsgemäßen Verfahren werden schnell und mit hoher Ausbeute Perfluoralkane hergestellt, die im wesentlichen frei sind von Polyfluoralkanen. Sie lassen sich zu allen jenen Zwecken anwenden, für welche derartige Perfluoralkane üblicherweise verwendet werden, beispielsweise auch im medizinischen Bereich.

Die folgenden Beispiele sollen die Erfindung weiter erläutern, ohne sie in ihrem Umfang einzuschränken.

### Beispiel 1:

### Reinigung von Perfluoralkanen

Ausgangsmaterial: Eingesetzt wurde ein Gemisch von folgender Zusammensetzung: Perfluorpentan ca. 41 %; Perfluorhexan ca. 42 %; Perfluorheptan ca. 16 %; polyfluorierte Alkane (wasserstoffhaltig, nicht näher identifiziert) < 0,5 % (alle Angaben in GC-Flächen-%). Ein solches Gemisch wird beispielsweise bei der elektrochemischen Fluorierung von Carbonsäuren durch Abbau bzw. Umlagerung der Ausgangsverbindungen bzw. Reaktionszwischenstufen oder bei der Perfluorierung entsprechender Alkane mittels bekannter Methoden erhalten.
In 200 g des angegebenen Gemisches wurde 1 g NaF suspendiert. Die Suspension wurde in einen Autoklaven eingegeben, bei Raumtemperatur mit je 1 bar N₂ und F₂ beaufschlagt, d. h. mit einem Reaktivgas, das 50 Vol.-% Stickstoff und 50 Vol.-% Fluor enthielt, und 7 Stunden bei 100 °C und dem sich einstellenden autogenen Druck (ca. 5 bis 6 bar abs) innig gerührt. Der Autoklav wurde entspannt. Dabei gast überschüssiges Reaktivgas aus. Feststoffe (NaF und seine Additionsverbindungen mit HF) wurden abfiltriert. Im NMR-Spektrum der Lösung konnten keine C-H-Resonanzen mehr nachgewiesen werden. Die gaschromatographische Analyse der Gasphase lieferte einen CF₄-Gehalt von lediglich 0,2 Flächen-% und keinen Hinweis auf höhere Homologe als Zersetzungsprodukte.
Das Beispiel belegt, daß man im wesentlichen ohne Bildung von Zersetzungsprodukten ein Perfluoralkan-Gemisch erzeugen kann, das im wesentlichen frei ist von wasserstoffhaltigen Fluorverbindungen.

### Beispiel 2:

### Reinigung von Perfluoralkanen ohne Zusatz von NaF

Beispiel 1 wurde wiederholt, diesmal wurde jedoch kein NaF zugegeben. Es wurden Ergebnisse wie in Beispiel 1 erhalten. Allerdings wurde zusätzlich Fluorwasserstoff im Gemisch nachgewiesen.
Die Aufarbeitung kann durch Zusatz von HF-Fängern erfolgen.
Die nach den Beispielen 1 und 2 erhaltenen Perfluoralkan-Gemische eignen sich beispielweise zur Verwendung als Lösungsmittel.

## Patentansprüche

1. Verfahren zur Herstellung von Perfluoralkanen, die im wesentlichen frei sind von Polyfluoralkanen, durch Umsetzung von Gemischen, welche Perfluoralkane und Polyfluoralkane enthalten, mit einem Reaktivgas ausgewählt aus der Gruppe umfassend elementares Fluor und ein Fluor/Inertgas-Gemisch, dadurch gekennzeichnet, daß man ein Reaktivgas mit einem Gehalt von 40 bis 100 Vol.-% Fluor und 0 bis 60 Vol.-% Inertgas einsetzt und die Umsetzung bei einem Druck von 4 bis 12 bar (abs.) und einer Temperatur von 75 - 120 °C durchführt.

2. Verfahren nach Anspruch 1, dadurch gekennzeichnet, daß man Gemische einsetzt, die max. 0,5 Mol.-% Polyfluoralkane enthalten.

3. Verfahren nach einem der vorhergehenden Ansprüche, dadurch gekennzeichnet, daß man in Anwesenheit einer zur Absorption von HF ausreichenden Menge von KF oder NaF arbeitet.

4. Verfahren nach Anspruch 1, 2, oder 3, dadurch gekennzeichnet, daß man die Umsetzung mit einem Reaktivgas bestehend aus 40 bis 60 Vol.-% Fluor und 60 bis 40 Vol.-% Inertgas und bei einem Druck von 5 bis 10 bar (abs.) und einer Temperatur von 80 bis 120 °C durchführt.

5. Verfahren nach Anspruch 4, dadurch gekennzeichnet, daß man die Umsetzung bei einer Temperatur von 80 bis 100 °C durchführt.

6. Verfahren nach einem der vorhergehenden Ansprüche, dadurch gekennzeichnet, daß man das Verfahren zur Herstellung von linearen oder verzweigten, aliphatischen Perfluoralkanen mit 5 bis 7 C-Atomen einsetzt.

7. Verfahren nach einem der vorhergehenden Ansprüche, dadurch gekennzeichnet, daß man die Umsetzung während einer Zeitdauer von 5 bis 10 Stunden durchführt.

## Claims

1. A method for the preparation of perfluoroalkanes which are substantially free of polyfluoroalkanes, by reacting mixtures which contain perfluoroalkanes and polyfluoroalkanes with a reactive gas selected from the group comprising elemental fluorine and a fluorine/inert gas mixture, characterised in that a reactive gas having a content of 40 to 100% by volume fluorine and 0 to 60% by volume inert gas is used and the reaction is performed at a pressure of 4 to 12 bar (abs.) and a temperature of 75 - 120°C.

2. A method according to Claim 1, characterised in that mixtures are used which contain at most 0.5 mole percent polyfluoroalkanes.

3. A method according to one of the preceding Claims, characterised in that one operates in the presence of a quantity of KF or NaF sufficient for absorbing HF.

4. A method according to Claim 1, 2 or 3, characterised in that the reaction is performed with a reactive gas consisting of 40 to 60% by volume fluorine and 60 to 40% by volume inert gas and at a pressure of 5 to 10 bar (abs.) and a temperature of 80 to 120°C.

5. A method according to Claim 4, characterised in that the reaction is performed at a temperature of 80 to 100°C.

6. A method according to one of the preceding Claims, characterised in that the method is used for the preparation of linear or branched, aliphatic perfluoroalkanes having 5 to 7 C atoms.

7. A method according to one of the preceding Claims, characterised in that the reaction is performed over a period of 5 to 10 hours.

## Revendications

1. Procédé pour la fabrication d'alcanes perfluorés essentiellement sans alcanes polyfluorés, obtenus par réaction de mélanges contenant des alcanes perfluorés et des alcanes polyfluorés avec un gaz de réaction du groupe comprenant le fluor élémentaire et un mélange de fluor et de gaz inerte, caractérisé par l'emploi d'un gaz de réaction dont le volume se compose de 40 à 100 % de fluor et de 0 à 60 % de gaz inerte, la réaction ayant lieu à une pression de 4 à 12 bars (absolut) et à une température de 75 - 120 °C.

2. Procédé selon la revendication 1, caractérisé en ce que l'on utilise des mélanges contenant au maximum 0,5 Mol.-% d'alcanes polyfluorés.

3. Procédé selon l'une des revendications précédentes caractérisé en ce que l'on procède en présence d'une quantité de KF ou NaF suffisante pour absorber le HF.

4. Procédé selon les revendications 1, 2 ou 3 caractérisé en ce que l'on effectue la réaction avec un gaz de réaction dont le volume se compose de 40 à 60 % de fluor et de 60 à 40 % de gaz inerte, la réaction ayant lieu à une pression de 5 à 10 bar (absolut) et une température de 80 à 120 °C.

5. Procédé selon l'une la revendication précédentes, caractérisé en ce que l'on effectue la réaction à une température de 80 à 100 °C.

6. Procédé selon l'une des revendications précédentes, caractérisé en ce que l'on utilise le procédé pour fabriquer des alcanes perfluorés aliphatiques linéaires ou ramifiés de 5 à 7 atomes de C.

7. Procédé selon l'une des revendications précédentes, caractérisé en ce que l'on poursuit la réaction pendant une durée de 5 à 10 heures.
